(19) **Europäisches Patentamt**

**European Patent Office**

**Office européen des brevets**

(11) **EP 1 138 665 B1**

(12) **FASCICULE DE BREVET EUROPEEN**

(45) Date de publication et mention
de la délivrance du brevet:
**10.09.2003 Bulletin 2003/37**

(51) Int Cl.⁷: **C07C 209/36**

(21) Numéro de dépôt: **01400779.3**

(22) Date de dépôt: **27.03.2001**

(54) **Hydrogénation continue de composés nitrés aromatiques utilisant un catalyseur à faible teneur en aluminium**

Kontinuierliche Hydrierung von aromatischen Nitroverbindungen unter Verwendung eines Katalysators mit geringem Gehalt an Aluminium

Continuous hydrogenation of aromatic nitro compounds using a catalyst with low aluminium content

(84) Etats contractants désignés:
**AT BE CH CY DE DK ES FI FR GB GR IE IT LI LU MC NL PT SE TR**

(30) Priorité: **28.03.2000 FR 0003903**

(43) Date de publication de la demande:
**04.10.2001 Bulletin 2001/40**

(73) Titulaire: **RHODIA CHIMIE**
**92512 Boulogne Billancourt Cedex (FR)**

(72) Inventeur: **Marion, Philippe**
**69390 Vernaison (FR)**

(74) Mandataire: **Wattremez, Catherine et al**
**Rhodia Services,**
**Direction de la Propriété Industrielle,**
**40, rue de la Haie-Coq**
**93306 Aubervilliers Cedex (FR)**

(56) Documents cités:
**FR-A- 1 290 268       GB-A- 821 220**
**US-A- 4 792 626**

• **EUGEN MÜLLER AND OTTO BAYER: "Methoden der Organischen Chemie (Houben-Weyl) Vol. VII/3b" , HOUBEN-WEYL METHODEN DER ORGANISCHEN CHEMIE,GEORGE THIEME VERLAG, STUTTGART,DE, VOL. IV, NR. 2, PAGES 171-174 XP002156244 * page 173, ligne 7 - ligne 8 ***

**Description**

La présente invention a pour objet un procédé d'hydrogénation catalytique effectuée en continu, de composés nitrés aromatiques mettant en oeuvre un catalyseur à faible teneur en aluminium ; les conditions de mises en oeuvre de l'hydrogénation étant telles que le temps de séjour du catalyseur est d'au moins trois jours.

Les procédés d'hydrogénation catalytique de composés nitrés aromatiques sont connus depuis de nombreuses années. Ces procédés comprennent en général habituellement deux zones et des périphériques. La première zone est celle dans laquelle la réaction d'hydrogénation proprement dite est effectuée ; la deuxième zone est celle dans laquelle le catalyseur est séparé du mélange réactionnel, et les périphériques désignent les tuyauteries et appareils (bacs de stockage, bacs de recyclage, pompes, etc. ) par lesquelles le mélange réactionnel et/ou les réactifs transitent, ainsi que la ou les zones de préparation du mélange réactionnel. Il est à noter que dans cette zone de préparation, on effectue en général un mélange des composés nitrés, de solvant frais s'il est utilisé, de catalyseur frais ainsi qu'une suspension de catalyseur provenant de la zone de réaction puis de séparation. Il n'est pas nécessaire que les zones de réaction et de séparation soient séparées ; en effet, elles peuvent exister dans un même appareillage, comme par exemple avec l'emploi d'un réacteur-décanteur. La réaction d'hydrogénation est une réaction très rapide et très exothermique qui est habituellement conduite en réacteur agité ou dans un réacteur à boucle de recirculation (loop reactor). Le procédé de séparation du catalyseur de l'hydrogénat peut être réalisé de diverses façons, comme la filtration tangentielle, la filtration frontale ou la décantation.

Habituellement, les procédés d'hydrogénation de composés nitrés aromatiques sont mis en oeuvre avec des catalyseurs comprenant au moins du nickel et de l'aluminium. Bien souvent, le catalyseur mis en oeuvre est de type nickel de Raney. Ce catalyseur peut être éventuellement dopé par divers métaux, comme le fer et/ou le chrome, notamment. Ces catalyseurs sont obtenus en effectuant un traitement basique d'un alliage comprenant le métal catalyseur, en l'occurrence le nickel, l'aluminium et éventuellement le ou les métaux dopants.

Le document S-4 792 626 décrit un catalyseur de type Nickel de Raney, modifié, ne contenant pas d'Aluminium, ou contenant de l'aluminium en teneur de 1 à 10% en poids. Le catalyseur est utilisé pour l'hydrogénation de composés nitrés aromatiques en amines aromatiques, et permet d'éviter l'utilisation d'un solvant. Le temps de séjour du catalyseur dans les installations n'est pas mentionné.

Plus classiquement, les procédés industriels développés mettent en jeu des catalyseurs de Raney dont les teneurs en aluminium résiduel sont relativement élevées, de l'ordre de 7 à 15 % en poids du catalyseur.

L'intérêt de tels catalyseurs est loin d'être remis en question dans la présente invention. Simplement, on a constaté que ces derniers présentent quelques inconvénients lorsqu'ils sont employés dans des conditions telles que la réaction est réalisée en continu, avec des temps de séjour du catalyseur élevé. Les mêmes limitations existent si ces catalyseurs sont mis en oeuvre dans des conditions de productivité importante.

En effet, dans ces conditions, l'oxydation parasite du nickel présent dans le catalyseur devient importante. Il apparaît alors dans le temps un dépôt très dur sur les parois du réacteur, du décanteur et/ou des périphériques au sens indiqué auparavant. Ce dépôt, que les inventeurs ont identifié comme possédant une structure d'aluminate de nickel, par exemple d'un hydroxyde double lamellaire du type de la takovite, est dû à cette importante oxydation parasite du nickel et à la présence concomitante d'oxyde d'aluminium hydraté.

Ce dépôt crée un blindage sur les parois du réacteur, du décanteur et des périphériques, ce qui diminue la productivité du procédé alors que le but recherché était justement de l'augmenter.

Il est donc nécessaire, à intervalles réguliers plus ou moins rapprochés, d'arrêter l'installation ou la zone concerné(e), et de nettoyer. Mais cette opération est très fastidieuse car elle nécessite l'emploi de moyens relativement puissants pour éliminer ce dépôt, comme par exemple l'emploi de lances à haute pression.

La présente invention a donc pour but de résoudre cet inconvénient, ou tout au moins d'en diminuer fortement les nuisances.

Ainsi, la présente invention a pour objet un procédé d'hydrogénation catalytique de composés aromatiques nitrés comprenant une zone de réaction, une zone de séparation et des périphériques, dans lequel l'hydrogénation est réalisée en continu, avec un catalyseur comprenant au moins du nickel et éventuellement de l'aluminium ; la teneur en aluminium total, s'il est présent, dans le catalyseur étant d'au plus 5,5 % en poids du catalyseur et le temps de séjour du catalyseur dans les zones de réaction, de séparation et des périphériques, étant d'au moins trois jours.

Par aluminium total, on entend la somme de l'aluminium (0) et de l'aluminium (+III).

On a constaté qu'en effectuant l'hydrogénation dans de telles conditions et en mettant en oeuvre un catalyseur comprenant dès le début de la réaction d'hydrogénation, une teneur en aluminium total beaucoup plus faible que celle présente dans les catalyseurs développés à l'échelle industrielle, voire nulle dans certains cas, l'apparition de l'aluminate de nickel est fortement ralentie, voire évitée.

Ainsi, la présente invention a-t-elle pour deuxième objet l'utilisation de catalyseur comprenant au moins du nickel et éventuellement de l'aluminium, la teneur en aluminium total, s'il est présent, étant d'au plus 5,5% en poids, dans le but de limiter l'apparition d'aluminates de nickel lors de la mise en oeuvre de réaction d'hydrogénation catalytique de composés nitrés aromatiques effectuée en continu.

Le ralentissement (ou la limitation) de l'apparition d'aluminate de nickel peut notamment être observé grâce à l'augmentation d'un facteur d'au moins 1,5, de préférence d'au moins 2, de la durée d'exploitation du procédé entre deux arrêts. Il est à noter que ces arrêts sont rendus nécessaires car le dépôt d'aluminate de nickel empêche alors la bonne marche de l'installation (diminution importante de la productivité, bouchage de conduites, notamment). Il est à noter que lorsque la teneur en aluminium initial dans le catalyseur est faible voire nulle, il est possible de ne pas avoir d'arrêt entre deux arrêts réglementaires.

De plus, et de manière surprenante, l'emploi d'un tel catalyseur ne dégrade pas de manière significative les performances de la réaction, notamment le rendement et la consommation en catalyseur. Il est même remarquable de noter que l'emploi de ce type de catalyseur peut contribuer à augmenter les performances de la réaction.

A titre illustratif, le procédé mis en oeuvre dans les conditions de l'invention permet d'obtenir un rendement en composé hydrogéné (donc en amine aromatique) d'au moins 99,5 % en poids.

Le catalyseur mis en oeuvre dans le procédé est particulièrement adapté pour permettre d'atteindre des productivités aussi élevées que 2 moles d'hydrogène transformées par heure et par gramme de catalyseur, voire jusqu'à 3 moles d'hydrogène transformées par heure et par gramme de catalyseur. Plus particulièrement, les productivités atteintes par le procédé selon l'invention sont comprises entre 0,1 et 3 moles d'hydrogène transformées/heure/gramme de catalyseur, de préférence entre 0,2 et 2 moles d'hydrogène transformées/heure/gramme de catalyseur.

En outre, le procédé selon l'invention permet d'utiliser dans cette réaction, des catalyseurs comprenant du nickel provenant d'autres procédés d'hydrogénation, comme par exemple ceux mis en oeuvre pour l'hydrogénation de composés comprenant des fonctions nitrées ou nitriles ou encore, provenant de la régénération de catalyseurs mis en oeuvre dans le procédé même.

Enfin, on a constaté que les catalyseurs mis en oeuvre dans le procédé selon l'invention, même dans des conditions de productivité importante, étaient désactivés moins rapidement que les catalyseurs classiques employés dans les mêmes conditions. En effet, le bouchage des pores des catalyseurs présentant une faible teneur en aluminium est moins rapide.

Mais d'autres caractéristiques et avantages de la présente invention apparaîtront plus clairement à la lecture de la description et des exemples qui vont suivre.

Ainsi que cela a été indiqué auparavant, le procédé selon l'invention concerne l'hydrogénation de composés nitrés aromatiques, en présence d'un catalyseur comprenant du nickel et de l'aluminium.

Selon une caractéristique de l'invention, le catalyseur présente une teneur en aluminium total d'au plus 5,5 % en poids du catalyseur. Il est à noter que le catalyseur présente cette teneur maximale en aluminium initialement, c'est-à-dire avant d'être engagé dans la réaction d'hydrogénation.

Selon un premier mode de réalisation particulier de l'invention, la teneur totale en aluminium est supérieure ou égale à 1 % en poids par rapport au poids total de catalyseur.

De préférence, la teneur en aluminium total est comprise entre 1 et 5,5 % en poids par rapport au poids total de catalyseur. De manière encore plus avantageuse, la teneur totale en aluminium dans le catalyseur est comprise entre 2 et 5 % en poids par rapport au poids total du catalyseur.

Selon une première variante de ce premier mode de réalisation de l'invention, le catalyseur est obtenu à partir d'un alliage à base de nickel, d'aluminium et éventuellement d'au moins un dopant (alliage de Raney).

Parmi les dopants classiques dans le domaine, on peut citer les métaux des colonnes IVA, VA, VIA, VIII de la classification périodique des éléments (supplément au bulletin de la société chimique de France, N°1 - Janvier 1966). De préférence, le dopant est choisi parmi le titane, le fer, le chrome, ou leur mélange.

Des catalyseurs de ce type peuvent provenir de différentes sources.

Selon une première source envisageable, on utilise un catalyseur provenant du traitement alcalin d'un alliage comprenant de l'aluminium, du nickel et éventuellement au moins un dopant ; la nature de l'alliage et/ou les conditions de fabrication de l'alliage et/ou les conditions de réalisation du traitement alcalin ayant pour conséquence que la teneur en aluminium total ne dépasse pas 5,5 % en poids du catalyseur.

Le traitement alcalin peut être, de manière avantageuse, réalisé comme suit

Tout d'abord, le composé alcalin est en général un hydroxyde de métal alcalin, comme le lithium, le sodium, le potassium, le césium, le rubidium.

Ce traitement peut être par exemple, réalisé avec un excès de composé alcalin par rapport à l'aluminium. Plus particulièrement, on peut mettre en oeuvre ledit composé alcalin dans une quantité telle que le rapport molaire de composé alcalin au nombre de moles d'aluminium présent est compris entre 1,5 et 10. De préférence, ledit rapport est compris entre 1,5 et 3.

Par ailleurs, on met en oeuvre; de manière classique, le composé alcalin sous la forme d'une solution aqueuse. Plus particulièrement la concentration du composé alcalin au début du traitement, est comprise entre 10 et 50 % en poids. De préférence, ladite concentration est de l'ordre de 15 à 30 % en poids.

De manière avantageuse et pour des raisons de sécurité, le traitement alcalin a lieu dans des conditions telles que la teneur en hydrogène dans la phase gazeuse reste inférieure à la limite inférieure d'explosivité du mélange air/hydrogène.

De telles conditions peuvent être obtenues, par exemple, en effectuant ledit traitement sous un balayage d'air ou encore d'un gaz inerte, comme entre autres, l'azote.

Il est de plus à noter que la pression à laquelle est mis en oeuvre le traitement alcalin est plus particulièrement supérieure ou égale à la pression de vapeur saturante du milieu liquide, à la température du traitement alcalin.

Rappelons que ce milieu liquide comprend entre autres, de l'eau, les sels d'aluminium dissous, le composé alcalin.

De plus, le terme pression de vapeur saturante désigne la pression partielle du milieu liquide à la température considérée.

En outre, la température à laquelle on effectue le traitement alcalin est de préférence comprise entre 50 et 130°C. De manière avantageuse, ledit traitement est effectué à une température comprise entre 60 et 90°C.

Il est précisé que la température du traitement peut évoluer durant le traitement alcalin. Plus particulièrement, il peut être avantageux d'augmenter la température en fin de traitement.

Quant à la durée du traitement alcalin, elle est plus particulièrement comprise entre 1 et 12 heures. Habituellement, cette durée est comprise entre 1 et 3 heures.

Il est enfin à noter que les conditions précitées sont choisies de telle manière que l'aluminium reste sous une forme soluble comme un aluminate, durant le traitement alcalin afin de pouvoir être séparé du solide restant après le traitement.

Une première possibilité consiste à utiliser un alliage précurseur du catalyseur présentant une forte teneur en aluminium. Par exemple, des-alliages présentent une teneur en aluminium supérieure à 50 % en poids de l'alliage peuvent convenir à l'invention.

Plus particulièrement, l'alliage comprend une teneur en aluminium inférieure ou égale à 70 % en poids de l'alliage, de préférence comprise entre 55 et 70 % en poids d'aluminium. Notons que l'on ne sortirait pas du cadre de la présente invention en utilisant des alliages dont la teneur en aluminium serait supérieure à 70 %. Simplement, l'utilisation de ce type d'alliage peut engendrer des coûts supplémentaires inutiles lors de son traitement (coût en matière première (composé alcalin, coût de traitement des effluents).

Selon un mode de réalisation avantageux de cette première possibilité, et si un dopant est présent dans l'alliage précurseur, on préfère effectuer le traitement sur un alliage dont la teneur en dopant ne dépasse pas 5 % en poids de l'alliage, et de préférence ne dépasse pas 3 % en poids de l'alliage.

Une deuxième possibilité consiste à réaliser le traitement alcalin de l'alliage précurseur dans des conditions plus poussées que celles indiquées pour le traitement alcalin décrit auparavant. Ainsi, parmi les conditions énoncées plus haut, il suffit qu'au moins l'une des conditions soit plus forte, pour considérer qu'il s'agit d'un traitement plus poussé. Par exemple, ces conditions peuvent consister en un rapport molaire composé alcalin / Al plus élevé, et/ou une concentration initiale du composé alcalin plus importante, et/ou une température maintenue durant le traitement ou température en fin de traitement plus élevée, et/ou une durée plus longue.

Selon un mode particulier de réalisation de cette deuxième possibilité, on peut aussi effectuer un traitement alcalin supplémentaire, c'est-à-dire effectuer au moins un autre traitement alcalin sur le produit issu d'un premier traitement alcalin d'un alliage, de manière à encore diminuer la teneur en aluminium résiduel.

Ainsi, on peut envisager réaliser le premier traitement alcalin dans des conditions classiques, le second dans des conditions plus poussées, comme indiqué ci-dessus.

Il est de même possible de mettre en oeuvre le second traitement dans des conditions similaires à celles du premier traitement.

Une troisième possibilité consiste à mettre en oeuvre un catalyseur à partir d'un alliage ayant subi un traitement particulier lors de sa fabrication. Ainsi, il est possible de mettre en oeuvre des catalyseurs obtenus à partir d'un alliage ayant subi une étape de recuit.

Plus particulièrement selon cette troisième possibilité, le lingot d'alliage est soumis à un traitement thermique à une température restant inférieure à la température péritectique conduisant à la formation de NiAl3. Plus particulièrement, la température mise en oeuvre dans ce traitement thermique est comprise entre 650 et 850°C.

Avantageusement, ce traitement est réalisé sous une atmosphère inerte.

Ce traitement permet de reconfigurer la structure de l'alliage et favorise l'apparition de phases riches en aluminium, comme NiAl3 ou la phase eutectique, par exemple. Or l'aluminium peut facilement être éliminé de ce type de phases, lors du traitement alcalin permettant d'accéder au catalyseur proprement dit.

Bien évidemment, on ne sortirait pas du cadre de la présente invention en mettant en oeuvre une combinaison des trois possibilités précitées.

Une deuxième source possible de catalyseur provient du recyclage de catalyseurs ayant été utilisés dans des procédés d'hydrogénation, différents ou non de celui dans lequel le catalyseur est mis en oeuvre.

A titre d'illustration, les catalyseurs de type nickel de Raney, dopés ou non, mis en oeuvre dans des hydrogénations de composés comprenant des fonctions nitrées ou nitriles peuvent convenir à cette variante. L'usure des catalyseurs lors de ces hydrogénations se traduit entre autres par une forte baisse de la teneur en aluminium résiduel.

Préalablement à leur emploi dans le procédé selon l'invention, le catalyseur est, de manière avantageuse, soumis à un premier lavage à l'eau de manière à éliminer toute trace des composés organiques présents lors du procédé antérieur. Le catalyseur résultant subit ensuite une étape de régénération consistant effectuer au moins un lavage alcalin. Cette opération a pour but d'éliminer tout ou partie de l'aluminium oxydé présent dans le catalyseur.

Les conditions détaillées précédemment pour les traitements alcalins peuvent être mises en oeuvre dans ce type de lavage.

Cependant, selon un mode de réalisation avantageux de cette variante, ledit lavage alcalin est effectué à une température inférieure ou égale à 150°C, bien que des températures plus élevées soient envisageables, selon le type d'appareillage mis en oeuvre ainsi que les sources d'énergie disponibles. Plus particulièrement, la température est supérieure ou égale à 50°C, de préférence comprise entre 80 et 140°C.

Le lavage alcalin peut être réalisé sous pression atmosphérique ou sous pression, selon les caractéristiques des appareils employés. Il est à noter qu'il n'y a pas d'avantages importants à effectuer le traitement à des pressions totales supérieures à $30.10^5$ Pa.

En ce qui concerne le composé alcalin, celui-ci peut être employé à une concentration inférieure ou égale à 35 % en poids dans l'eau.

Cette étape de régénération peut aussi être conduite avantageusement sous pression d'hydrogène pour éventuellement réduire à nouveau une partie du nickel oxydé.

Dans un tel cas, le traitement est effectué plus particulièrement avec une pression partielle en hydrogène d'au moins $10^5$ Pa (1 bar). La pression partielle en hydrogène peut être aussi élevée que possible, selon le matériel mis en oeuvre. Cependant, avantageusement, cette pression partielle en hydrogène est inférieure ou égale à $30.10^5$ Pa (30 bar). La pression totale, dans le cas d'un traitement sous hydrogène est de manière avantageuse comprise entre 6 et $30.10^5$ Pa (6 - 30 bar).

Une deuxième variante du premier mode de réalisation de l'invention est constituée par des catalyseurs comprenant du nickel, de l'aluminium et un support.

Le nickel peut éventuellement être combiné à au moins un métal dopant comme ceux appartenant aux colonnes IVA, VA, VIA, VIII-de la classification périodique des éléments, de préférence, parmi le titane, le fer, le chrome, ou leur mélange.

Ledit support est plus particulièrement choisi de telle sorte qu'il soit stable dans les conditions de réaction.

Plus particulièrement, le support comprend de l'aluminium (plus particulièrement sous forme d'alumine) combiné à au moins un oxyde choisi parmi les oxydes de zirconium, de titane, de silicium, seuls ou en mélanges.

Il est à noter que le catalyseur est tel que la teneur totale en aluminium du catalyseur quel que soit son degré d'oxydation remplit les conditions indiquées auparavant, à savoir qu'elle ne dépasse pas 5,5 % en poids total du catalyseur.

La quantité de support est plus particulièrement comprise entre 25 et 80 % en poids de catalyseur, de préférence entre 30 et 65 % en poids de catalyseur.

Ce type de catalyseur est habituellement obtenu par précipitation d'un sel de nickel et éventuellement d'au moins un sel d'un métal dopant, sur le support. Le catalyseur est ensuite séparé par filtration, par exemple, puis séché et calciné à une température suffisante pour transformer l'hydroxyde de nickel et éventuellement le sel ou l'hydroxyde du métal dopant, en oxydes. Une fois cette étape réalisée, le catalyseur subit un traitement durant lequel une partie du nickel est réduite. Cette réduction est généralement effectuée à haute température sous une atmosphère réductrice, comme l'hydrogène.

Il serait possible de mettre en oeuvre dans la réaction selon l'invention un catalyseur supporté tel qu'il vient d'être décrit, ayant au préalable été utilisé dans un autre procédé d'hydrogénation. Dans un tel cas, il est préférable, avant toute utilisation dudit catalyseur usé dans le procédé selon l'invention, de mettre en oeuvre une étape de lavage à l'eau, voire un lavage alcalin, de manière à éliminer toute trace de composés organiques provenant du procédé antérieur. Après ce lavage, il peut être recommandé d'effectuer un traitement de réduction, de préférence, effectué à haute température sous une atmosphère réductrice, comme l'hydrogène.

Selon un second mode de réalisation particulier de l'invention, le catalyseur comprend du nickel mais ne comprend pas d'aluminium.

Selon ce mode de réalisation, le catalyseur mis en oeuvre comprend un support qui est exempt d'aluminium. Ce qui a été dit auparavant concernant les catalyseurs supportés comprenant de l'aluminium (préparation ; origine (neuf, recyclé), traitement du catalyseur recyclé avant son introduction dans le procédé d'hydrogénation selon l'invention) reste valable pour ce mode de réalisation, étant entendu que l'aluminium n'est pas présent dans ce mode de réalisation particulier.

Le catalyseur qui vient d'être détaillé est donc mis en oeuvre dans une réaction d'hydrogénation catalytique de composés aromatiques nitrés, conduite en continu.

Selon une caractéristique importante du procédé selon l'invention, le temps de séjour du catalyseur est d'au moins 3 jours.

Au sens de la présente invention, le temps de séjour est déterminé comme suit :

$$\text{Tps séjour} = \frac{\text{Encours catalyseur}}{\text{Consommation catalyseur x Production composé aminé}}$$

Encours catalyseur : quantité de catalyseur dans les zones de réaction, séparation, périphériques ; exprimée en kg.

Consommation catalyseur : quantité de catalyseur consommé dans le procédé (quantité de catalyseur qui est purgée et qui n'est pas réinjectée dans le procédé par la suite) ; exprimée en kg de catalyseur purgé par tonne de composé aminé produit.

Production composé aminé : quantité de composé aminé produit, exprimée en tonne par jour.

Bien souvent, le temps de séjour du catalyseur dans ces zones est de l'ordre de plusieurs semaines.

Par composés aromatiques nitrés, on désigne notamment les composés comprenant au moins une fonction nitrée, et de préférence au moins deux fonctions nitrées, et au moins un motif aromatique en $C_6$-$C_{14}$, de préférence en $C_6$-$C_{10}$, substitué ou non par un ou plusieurs radicaux hydrocarbonés, saturés ou non, linéaires, cycliques ou ramifiés en $C_1$-$C_{10}$, et/ou un ou plusieurs radicaux hydroxyles.

Plus précisément, les radicaux hydrocarbonés précités, substituant éventuellement lesdits motifs aromatiques, peuvent être choisis parmi les radicaux alkyles, aryles, alkylaryles et arylalkyles en $C_1$-$C_{10}$, de préférence en $C_1$-$C_6$.

Comme motif aromatique, on peut notamment citer les noyaux benzénique et naphtalénique, substitués ou non par un ou plusieurs radicaux méthyle, éthyle, propyle, butyle, pentyle, hexyle et/ou leurs isomères.

Le procédé selon l'invention peut être mis en oeuvre en utilisant au moins un composé choisi parmi le mononitrobenzène, le dinitrobenzène, le mononitrotoluène, le dinitrotoluène, le paranitrocumène, l'orthonitrophénol.

Le procédé selon l'invention est très avantageusement mis en oeuvre avec un mélange d'isomères de dinitrotoluènes.

Les composés nitrés aromatiques peuvent être mis en oeuvre en présence d'un solvant desdits composés.

Selon une première variante, le solvant employé est choisi parmi les alcools aliphatiques ou les éthers cycliques, seuls ou en mélange

Plus particulièrement, on utilise à titre d'alcool aliphatique le méthanol, l'éthanol, le propanol, l'isopropanol, seuls ou en mélange.

A titre d'éther cyclique, on peut citer le dioxane, le tétrahydrofuranne, seuls ou en mélange.

Selon cette variante, les composés nitrés aromatiques, préalablement à l'introduction dans la zone de réaction, sont solubilisés dans le solvant ou le mélange de solvants précités.

La concentration en composés nitrés dans le solvant ou le mélange de solvants, peut varier dans un large domaine. On utilise cependant des mélanges monophasiques, c'est-à-dire pour lesquels la limite de solubilité du ou des composés nitrés n'est pas atteinte pour le solvant ou le mélange en question. Sans intention de se limiter, la gamme de concentration est inférieure ou égale à 25 % en poids.

Selon une seconde variante préférée de l'invention, le solvant employé est le produit de l'hydrogénation, à savoir un mélange amine/eau. Si cette variante est retenue, les composés nitrés aromatiques sont introduits à l'état liquide ou fondu.

Cette variante est intéressante en ce sens où elle ne dilue pas le réactif comme c'est le cas avec l'usage d'un solvant ou d'un mélange de solvants. Cela simplifie le procédé car cette variante s'affranchit de toute étape supplémentaire de séparation des produits du ou des solvants mis en oeuvre lors de la réaction, qui comporte des risques de pertes des produits hydrogénés.

La teneur instantanée dans la zone de réaction, en composés nitrés est maintenue la plus faible possible. Habituellement, on fait en sorte qu'elle ne dépasse pas 1000 ppm. Maintenir cette teneur en composés nitrés dans le réacteur est réalisé en réglant le débit d'alimentation en composés nitrés de telle sorte qu'ils soient réduits quasiment instantanément par le catalyseur dès leur introduction dans le réacteur. Le procédé selon l'invention est très bien adapté pour fonctionner à des productivités très élevées, c'est-à-dire que le composé nitré aromatique est alimenté presque au débit maximal absorbable par le couple réacteur/catalyseur.

L'hydrogène employé est plus particulièrement de l'hydrogène pur. Par pur, on entend un gaz comprenant au moins 99 % d'hydrogène, et plus particulièrement au moins 99,9 % d'hydrogène. Il est à noter que l'on ne sortirait pas du cadre de la présente invention en mettant en oeuvre la réaction d'hydrogénation avec de l'hydrogène dilué, bien que cela n'apporte pas d'avantage particulier à la réaction.

De préférence, l'hydrogène est alimenté dans la quantité stoechiométrique. Cependant, on ne sortirait pas du cadre de la présente invention en mettant en oeuvre l'hydrogénation avec un excès d'hydrogène par rapport à la stoechiométrie. De telles conditions peuvent être avantageuses pour satisfaire des critères d'hydrodynamiques, plus particulièrement pour optimiser le transfert gaz-liquide de certaines technologies.

La pression d'hydrogène dans le réacteur varie, de manière avantageuse, entre 5 et $70.10^5$ Pa (5 et 70 bar), de préférence entre 10 et $50.10^5$ Pa (10 et 50 bar).

L'alimentation en ce gaz est effectuée par tout moyen connu de l'homme du métier, permettant d'avoir une répartition homogène de ce gaz au sein du réacteur.

La réaction est de préférence effectuée dans un réacteur agité ou dans un réacteur à boucle de recirculation.

La température est plus particulièrement comprise entre 50 et 200°C.

A l'issue de la réaction d'hydrogénation, le mélange réactionnel est séparé du catalyseur par décantation ou filtration, selon les méthodes classiques.

Bien évidemment, la quantité de mélange réactionnel séparé correspond à la quantité de composé nitré, éventuellement en présence du solvant, introduite dans la zone de réaction ; ceci afin de maintenir des conditions de fonctionnement stables.

Le mélange réactionnel séparé du catalyseur est ensuite traité selon sa composition et son utilisation ultérieure.

Ainsi, dans le cas où la réaction est effectuée avec un solvant, le produit hydrogéné obtenu est séparé par distillation, par exemple. Il est à noter que selon le choix du solvant et l'étape ultérieure à laquelle seront soumis les produits hydrogénés, cette étape de séparation peut ne pas être nécessaire.

Le produit hydrogéné aromatique peut ensuite être engagé dans une réaction de phosgénation, après avoir été déshydraté, de manière à obtenir des isocyanates aromatiques, intermédiaires dans la production de polyuréthannes, ou bien encore, si nécessaire, distillé pour séparer les différents isomères obtenus.

Des essais concrets mais non limitatifs de l'invention vont maintenant être présentés.

EXEMPLE 1 (comparatif)

L'évaluation d'un nickel Raney standard non dopé contenant 8%pds d'aluminium total est faite en mettant en oeuvre deux tests (test 1 et test 2).

a) Test 1 :

On alimente en continu 50 g/h d'un mélange d'isomères de dinitrotoluène (DNT) dans un réacteur contenant 0,7 g de nickel de Raney à 150°C sous 20 bar d'hydrogène.

L'alimentation en DNT se poursuit jusqu'à ce que la teneur en DNT dans le réacteur dépasse 1000 ppm.

L'alimentation de l'hydrogène est guidée par l'avancement de la réaction pour rester à pression constante dans le réacteur.

On arrête la réaction au bout de 6 heures.

Le rendement de la réaction à t = 4 heures est voisin de 99%.

b) Test 2 :

Un échantillon du catalyseur initial (n'ayant pas subi l'hydrogénation) est introduit avec un mélange d'isomères de toluènediamine (TDA) et d'eau dans un tube de verre dit tube de Carius qui est scellé puis est laissé pendant 1 mois à 150°C sous pression autogène.

Au bout d'un mois, on ouvre le tube : le catalyseur est irrécupérable. Il est extrêmement dur.

Par diffraction des RX, la structure du catalyseur est un aluminate de nickel (takovite). Cette structure est la même que celle du dépôt (blindage) observé sur les parois des appareillages mis en oeuvre à l'échelle industrielle.

EXEMPLE 2

Le catalyseur employé est un nickel Raney récupéré d'une unité d'hydrogénation de nitrile contenant 3,5%pds d'aluminium total. Le catalyseur est tout d'abord lavé à l'eau puis à la soude diluée (1N) puis à nouveau à l'eau. Le catalyseur est alors évalué selon les mêmes tests que ceux décrits dans l'exemple 1.

a) Test 1 :

On reproduit le test 1 de l'exemple 1.

Les résultats sont les suivants :

On arrête alors la réaction au bout de 8 heures.
Le rendement de la réaction à t= 4h est de 99,2%.

b) Test 2 :

On reproduit le test 2 de l'exemple 1.
Les résultats sont les suivants

Au bout d'un mois, on ouvre le tube: le catalyseur est récupérable. Il est légèrement dur.
Par diffraction des RX, la structure du catalyseur est essentiellement la structure nickel. Elle contient une faible proportion d'aluminate de nickel (au moins 2 fois plus faible que celle du catalyseur de l'exemple 1).

EXEMPLE 3

Le catalyseur mis en oeuvre dans cet exemple est un catalyseur à base de nickel non dopé, supporté, contenant environ 2 %pds d'aluminium total sous forme d'alumine, 2 à 6% de $ZrO_2$,de la silice et du nickel avec une teneur en nickel total de 55%pds.
Le catalyseur est évalué selon les mêmes tests que ceux décrits dans l'exemple 1.

a) Test 1 :

On reproduit le test 1 de l'exemple 1.
Les résultats sont les suivants :

On arrête alors la réaction au bout de 9 heures.
Le rendement de la réaction à t= 4h est voisin de 99,2%.

c) Test 2 :

On reproduit le test 2 de l'exemple 1.
Les résultats sont les suivants :

Au bout d'un mois, quand on ouvre le tube, le catalyseur est récupérable. Il n'est pas dur.
Par diffraction des RX, la structure du catalyseur est essentiellement la même que celle observée sur le catalyseur neuf.

**Revendications**

1.   Procédé d'hydrogénation catalytique de composés aromatiques nitrés comprenant une zone de réaction, une zone de séparation et des périphériques, **caractérisé en ce que** l'hydrogénation est réalisée en continu, avec un catalyseur comprenant au moins du nickel et éventuellement de l'aluminium, **en ce que** la teneur en aluminium total, s'il est présent, dans le catalyseur est d'au plus 5,5 % en poids du catalyseur et **en ce que** le temps de séjour du catalyseur dans les zones de réaction, de séparation et des périphériques, est d'au moins trois jours.

2.   Procédé selon la revendication précédente, **caractérisé en ce que** la teneur en aluminium total est supérieure ou égale à 1 % par rapport au poids de catalyseur, et de préférence, comprise entre 2 et 5 % en poids par rapport à la même référence.

3.   Procédé selon l'une quelconque des revendications précédentes, **caractérisé en ce que** le catalyseur est obtenu à partir d'un alliage comprenant du nickel, de l'aluminium, et éventuellement au moins un dopant.

4.   Procédé selon la revendication précédente, **caractérisé en ce que** le ou les dopants sont choisis parmi les métaux des colonnes IVA, VA, VIA, VIII de la classification périodique des éléments, de préférence, parmi le titane, le fer, le chrome, ou leur mélange.

5.   Procédé selon l'une quelconque des revendications 1 à 2, **caractérisé en ce que** le catalyseur comprend un support.

6. Procédé selon la revendications précédente, **caractérisé en ce que** le support comprend de l'oxyde d'aluminium combiné à au moins un oxyde choisi parmi les oxydes de zirconium, de titane, de silicium, seuls ou en mélanges.

7. Procédé selon la revendication 1, **caractérisé en ce que** le catalyseur comprend un support exempt d'aluminium.

8. Procédé selon l'une quelconque des revendications précédentes, **caractérisé en ce que** l'hydrogénation est effectuée à une température comprise entre 50 et 200°C.

9. Procédé selon l'une quelconque des revendications précédentes, **caractérisé en ce que** l'hydrogénation est effectuée à une pression comprise entre 5 et $70.10^5$ Pa.

10. Procédé selon l'une quelconque des revendications précédentes, **caractérisé en ce que** les conditions de mises en oeuvre de la réaction sont telles que la productivité est d'au plus 3 moles d'hydrogène transformé par heure et par gramme de catalyseur, de préférence d'au plus 2 moles d'hydrogène transformé par heure et par gramme de catalyseur.

11. Procédé selon l'une quelconque des revendications précédentes, **caractérisé en ce que** le composé nitré aromatique est choisi parmi les composés comprenant au moins une fonction nitrée, et de préférence au moins deux fonctions nitrées, et au moins un motif aromatique en $C_6$-$C_{14}$, de préférence en $C_6$-$C_{10}$, substitué ou non par un ou plusieurs radicaux hydrocarbonés, saturés ou non, linéaires, cycliques ou ramifiés en $C_1$-$C_{10}$, et/ou un ou plusieurs radicaux hydroxyles.

12. Procédé selon l'une quelconque des revendications précédentes, **caractérisé en ce que** l'hydrogénation est réalisée en présence d'un solvant des composés nitrés aromatiques.

13. Procédé selon la revendication 12, **caractérisé en ce que** le solvant est choisi parmi les alcools aliphatiques ou les éthers cycliques, seuls ou en mélange.

14. Procédé selon la revendication 12, **caractérisé en ce que** l'on effectue la réaction en présence d'un solvant qui est le composé hydrogéné.


**Patentansprüche**

1. Verfahren zur katalytischen Hydrierung von aromatischen Nitroverbindungen, umfassend eine Reaktionszone, eine Trennzone und Peripheriegeräte, **dadurch gekennzeichnet, daß** die Hydrierung kontinuierlich mit einem Katalysator realisiert wird, der mindestens Nickel und gegebenenfalls Aluminium umfaßt, und daß der Gesamtgehalt an Aluminium, wenn es anwesend ist, in dem Katalysator höchstens 5,5 Gew.-% des Katalysators beträgt, und daß die Verweilzeit des Katalysators in den Zonen der Reaktion, der Trennung und in den Peripheriegeräten mindestens drei Tage beträgt.

2. Verfahren nach dem vorstehenden Anspruch, **dadurch gekennzeichnet, daß** der Gesamtgehalt an Aluminium höher oder gleich 1 % beträgt, bezogen auf das Gewicht des Katalysators, und vorzugsweise zwischen 2 und 5 Gew.-% liegt, bezogen auf die gleiche Referenz.

3. Verfahren nach irgendeinem der vorstehenden Ansprüche, **dadurch gekennzeichnet, daß** der Katalysator ausgehend von einer Legierung erhalten wird, die Nickel, Aluminium und gegebenenfalls mindestens ein Dotierungsmittel umfaßt.

4. Verfahren nach dem vorstehenden Anspruch, **dadurch gekennzeichnet, daß** das oder die Dotierungsmittel ausgewählt werden unter den Metallen der Reihen IVA, VA, VIA, VIII des Periodensystems der Elemente und vorzugsweise unter Titan, Eisen, Chrom oder ihren Mischungen.

5. Verfahren nach einem der Ansprüche 1 bis 2, **dadurch gekennzeichnet, daß** der Katalysator einen Träger umfaßt.

6. Verfahren nach dem vorstehenden Anspruch, **dadurch gekennzeichnet, daß** der Träger Aluminiumoxid umfaßt, kombiniert mit mindestens einem Oxid, ausgewählt unter den Oxiden von Zirkonium, Titan, Silicium, allein oder in Mischung.

**7.** Verfahren nach Anspruch 1, **dadurch gekennzeichnet, daß** der Katalysator einen Träger umfaßt, der frei von Aluminium ist.

**8.** Verfahren nach irgendeinem der vorstehenden Ansprüche, **dadurch gekennzeichnet, daß** die Hydrierung bei einer Temperatur zwischen 50 °C und 200 °C durchgeführt wird.

**9.** Verfahren nach irgendeinem der vorstehenden Ansprüche, **dadurch gekennzeichnet, daß** die Hydrierung bei einem Druck zwischen 5 und $70.10^5$ Pa durchgeführt wird.

**10.** Verfahren nach irgendeinem der vorstehenden Ansprüche, **dadurch gekennzeichnet, daß** die Bedingungen zur Durchführung der Reaktion derart sind, daß die Produktivität höchstens 3 Mol von umgewandeltem Wasserstoff pro Stunde und Gramm des Katalysators beträgt, vorzugsweise höchstens 2 Mol von umgewandeltem Wasserstoff pro Stunde und Gramm des Katalysators.

**11.** Verfahren nach irgendeinem der vorstehenden Ansprüche, **dadurch gekennzeichnet, daß** die aromatische Nitroverbindung ausgewählt wird unter den Verbindungen, die mindestens eine Nitrofunktion und vorzugsweise mindestens zwei Nitrofunktionen, und mindestens eine aromatische Struktureinheit mit 6 bis 14 Kohlenstoffatomen, vorzugsweise 6 bis 10 Kohlenstoffatomen umfassen, unsubstituiert oder substituiert durch einen oder mehrere gesättigte oder ungesättigte, lineare, cyclische oder verzweigte Kohlenwasserstoff-Reste mit 1 bis 10 Kohlenstoffatomen und/oder einen oder mehrere Hydroxylreste.

**12.** Verfahren nach irgendeinem der vorstehenden Ansprüche, **dadurch gekennzeichnet, daß** die Hydrierung in Anwesenheit eines Lösungsmittels für die aromatischen Nitroverbindungen realisiert wird.

**13.** Verfahren nach Anspruch 12, **dadurch gekennzeichnet, daß** das Lösungsmittel ausgewählt wird unter aliphatischen Alkoholen oder den cyclischen Ethern, allein oder in Mischung.

**14.** Verfahren nach Anspruch 12, **dadurch gekennzeichnet, daß** man die Reaktion in Anwesenheit eines Lösungsmittels durchführt, das die hydrierte Verbindung darstellt.

**Claims**

**1.** Process for the catalytic hydrogenation of nitrated aromatic compounds, comprising a reaction zone, a separation zone and peripherals, **characterized in that** the hydrogenation is carried out continuously, with a catalyst comprising at least nickel and possibly aluminium, **in that** the total aluminium content, if aluminium is present, in the catalyst is at most 5.5% by weight of the catalyst and **in that** the residence time of the catalyst in the reaction zone, separation zone and peripherals is at least three days.

**2.** Process according to the preceding claim, **characterized in that** the total aluminium content is greater than or equal to 1% with respect to the weight of catalyst, and preferably between 2 and 5% by weight with respect to the same reference.

**3.** Process according to either of the preceding claims, **characterized in that** the catalyst is obtained from an alloy comprising nickel, aluminium and possibly at least one dopant.

**4.** Process according to the preceding claim, **characterized in that** the dopant(s) is/are chosen from the metals of Groups IVA, VA, VIA, VIII of the Periodic Table of Elements, preferably from titanium, iron and chromium, or a mixture thereof.

**5.** Process according to either of claims 1 and 2, **characterized in that** the catalyst comprises a support.

**6.** Process according to the preceding claim, **characterized in that** the support comprises aluminium oxide combined with at least one oxide chosen from zirconium, titanium and silicon oxides, by themselves or as mixtures.

**7.** Process according to claim 1, **characterized in that** the catalyst comprises a support containing no aluminium.

**8.** Process according to any one of the preceding claims, **characterized in that** the hydrogenation is carried out at a temperature of between 50 and 200°C.

9. Process according to any one of the preceding claims, **characterized in that** the hydrogenation is carried out at a pressure of between 5 and $70 \times 10^5$ Pa.

10. Process according to any one of the preceding claims, **characterized in that** the conditions under which the reaction is carried out are such that the productivity is at most 3 mol of converted hydrogen per hour and per gram of catalyst, preferably at most 2 mol of converted hydrogen per hour and per gram of catalyst.

11. Process according to any one of the preceding claims, **characterized in that** the aromatic nitrated compound is chosen from compounds comprising at least one nitrated functional group, and preferably at least two nitrated functional groups, and at least one $C_6$-$C_{14}$, preferably $C_6$-$C_{10}$ aromatic unit which may or may not be substituted with one or more $C_1$-$C_{10}$ linear, cyclic or branched, saturated or unsaturated, hydrocarbon radicals and/or one or more hydroxyl radicals.

12. Process according to any one of the preceding claims, **characterized in that** the hydrogenation is carried out in the presence of a solvent for the aromatic nitrated compounds.

13. Process according to claim 12, **characterized in that** the solvent is chosen from aliphatic alcohols or cyclic ethers, by themselves or as a mixture.

14. Process according to claim 12, **characterized in that** the reaction is carried out in the presence of a solvent which is the hydrogenated compound.